Europäisches Patentamt

European Patent Office

Office européen des brevets

(11) Veröffentlichungsnummer: **0 312 787**
**A1**

## EUROPÄISCHE PATENTANMELDUNG

(21) Anmeldenummer: 88115441.3

(22) Anmeldetag: 21.09.88

(51) Int. Cl.⁴: **A61B 1/00 , A61B 1/30 , A61B 17/34**

(30) Priorität: 21.10.87 DE 8714069 U

(43) Veröffentlichungstag der Anmeldung:
26.04.89 Patentblatt 89/17

(84) Benannte Vertragsstaaten:
CH DE FR GB LI NL

(71) Anmelder: **Richard Wolf GmbH**
**Pforzheimer Strasse 32**
**D-7134 Knittlingen(DE)**

(72) Erfinder: **Bonnet, Ludwig**
**Jahnstrasse 28**
**D-7134 Knittlingen(DE)**

(74) Vertreter: **Wilcken, Thomas, Dipl.-Ing. et al**
**Musterbahn 1**
**D-2400 Lübeck(DE)**

(54) **Endoskopischer Instrumentensatz.**

(57) Der endoskopische Instrumentensatz zur Diagnose und Therapie im Bereich des Hydroscrotums besteht aus einer als Kanüle ausgebildeten Punktionsnadel (4), die mit mindestens einer nach außen abgestuften Erweiterung (6,7) im Abstand vom distalen Ende eines Schaftes (1) versehen ist und die gegen eine erste Optik (8) zur Diagnose oder erforderlichenfalls durch eine zweite Optik (9) mit Hilfsinstrumenten zur Therapie austauschbar ist.

Fig. 1

EP 0 312 787 A1

## Endoskopischer Instrumentensatz

Die Erfindung bezieht sich auf einen endoskopischen Instrumentensatz nach dem Oberbegriff des Anspruches 1.

Zur Diagnose und eventuellen Therapie des Hodens bei Auftreten von Fertilitätsstörungen, Hodentorsionen, Anomalien des Nebenhodens und des distalen Samenstranges, Tumoren oder dgl. ist es bisher erforderlich, das Scrotum (Hodensack) mittels einer Punktionsnadel zu punktieren, um einen Führungsdraht im Hydroscrotum zu placieren, über den zur Aufweitung des Punktionskanals Dilatatoren geführt werden müssen, um anschließend zur Diagnose und/oder Therapie einen Schaft mit einer Optik und etwaigen Hilfsinstrumenten in das Hydroscrotum einführen zu können. Dieses Vorgehen ist eine hohe Belastung für den Patienten.

Die Aufgabe der Erfindung besteht darin, das aufeinanderfolgende Einführen und Herausnehmen der Punktionsnadel, der Dilatatoren und des Schaftes durch einen einzigen Durchführungsvorgang zu ersetzen.

Diese Aufgabe wird nach der Erfindung durch die kennzeichnenden Merkmale des Anspruches 1 gelöst.

Durch diese Lösung wird nach erfolgter Punktion durch weiteres Einführen der Punktionsnadel in das Hydroscrotum eine Aufweitung des Punktionskanales erreicht und die Verwendung eines einzuführenden Führungsdrahtes sowie das Aufweiten des Punktionskanales mittels eines oder mehrerer Dilatatoren vermieden. Der im aufgeweiteten Punktionskanal verbleibende Schaft ermöglicht es nach dem Herausziehen der Punktionsnadel wahlweise eine Optik zur Diagnose oder eine zweite Optik zur Therapie mit Hilfsinstrumenten in das Hydroscrotum einzuführen.

Vorteilhaft geht man nach der Erfindung im einzelnen so vor, daß die als Punktionskanüle zum Zu- und Abführen einer Flüssigkeit ausgebildete Punktionsnadel im Abstand vom distalen Ende mit einer mindestens einfach nach außen abgestuften Erweiterung versehen ist, deren kegeliger Übergang entweder im Bereich des distalen Ende des Schaftes zu liegen kommt oder aus diesem über eine bestimmte Länge herausragt.

Durch diese Erweiterung, die wie schon erwähnt, nach außen auch mehrfach abgestuft sein kann, wird der Punktionskanal einmal oder nacheinander mehrfach bis etwa auf den Durchmesser des distalen Schaftendes aufgeweitet.

Die Erfindung wird nachstehend anhand der Zeichnung erläutert. Es zeigt:

Figur 1 einen axialen Längsschnitt durch den Instrumentenschaft mit der Punktionsnadel,

Figur 2 eine Seitenansicht des in Figur 1 gezeigten Schaftes mit einer durchgeführten Diagnoseoptik und

Figur 3 die zu Figur 2 gleiche Ansicht, jedoch mit ausgetauschter Optik für die Therapie.

Das Scrotoscop nach der Erfindung besteht aus einem Schaft 1 mit absperrbaren, am proximalen erweiterten Teil 1a angeordnetem Einführstutzen 2 sowie zwei sich gegenüberliegende Saug- und Spülanschlüsse 2a. Proximal ist der Schaft mit einem Spannverschluß 3 versehen, in dem eine durch den Schaft 1 hindurchzuführende Punktionsnadel 4 mit ihrem Kupplungskegel 3a festgelegt wird. Zum Anschließen eines mit einer Saug- und Spülpumpe verbundenen Schlauches weist die Nadel 4 am proximalseitigen Ende der Handhabe 5 ein Anschlußteil 5a auf.

Die Punktionsnadel 4 ist in einer ersten Ausführungsform mit einer im Abstand vom distalen Ende angeordneten Hülse 6 versehen, die durch einen kegeligen Übergang 7 das distale Nadelende im Durchmesser somit vergrößert, daß das distale Ende des Schaftes 1 die Hülse 6 mit nur geringem Spiel umgibt. Anstelle der durch mehrere Rohrteile zusammengesetzten Kanüle kann diese auch aus einem Stück gefertigt sein.

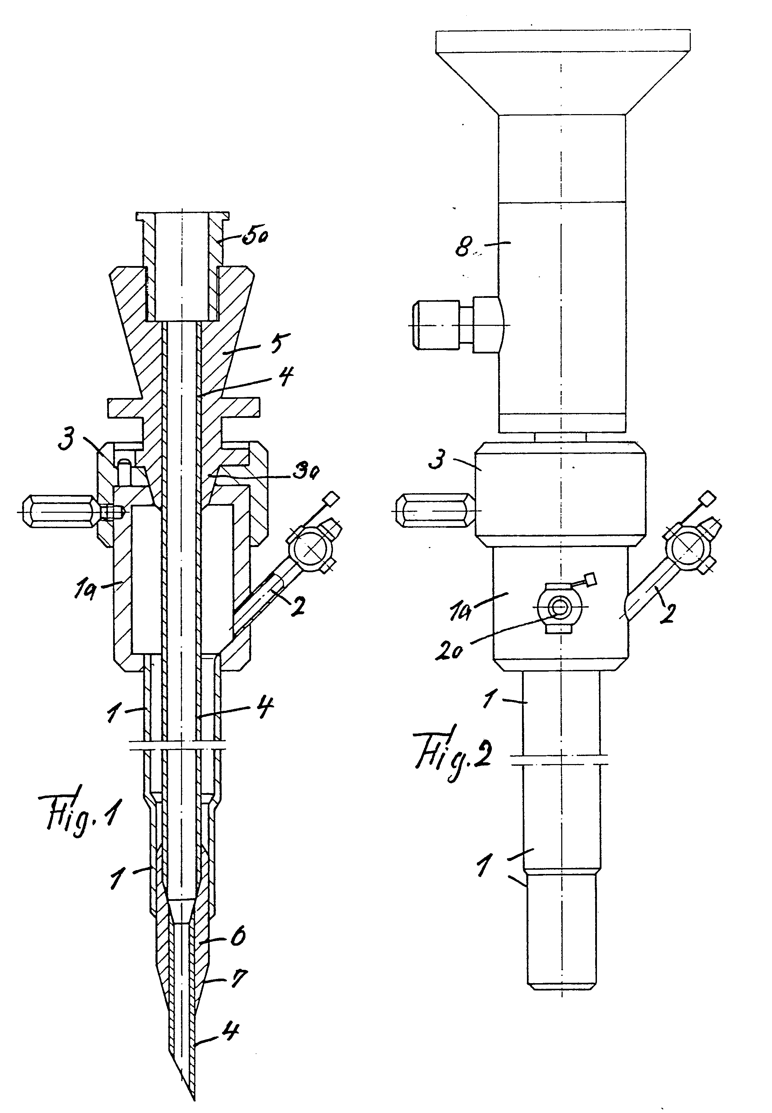

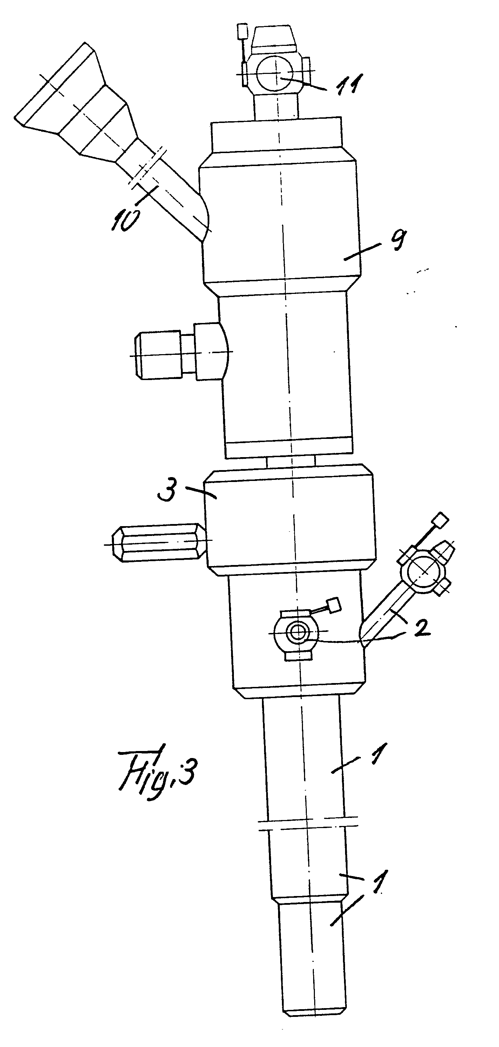

Nach erfolgter Punktion des Scrotums und Dilatation des Punktionskanales wird die Punktionsnadel 4,7,6 nach Lösen des Spannverschlusses 3 aus dem mit dem am distalen Ende in dem Punktionskanal verbleibenden Schaft 1 herausgezogen und durch eine Optik 8 nach Figur 2 zur Diagnose ersetzt. Nach der Diagnose kann die Optik 8 nach Figur 3 erforderlichenfalls auch durch eine Optik 9 mit proximal seitlicher Abwinkelung 10 ersetzt werden, die mit einem proximal bei 11 absperrbaren Kanal für durchzuführende Hilfsinstrumente versehen ist.

## Ansprüche

1. Endoskopischer Instrumentensatz zur Diagnose und Therapie im Bereich des Hydroscrotums, bestehend aus einer Punktionsnadel und einer Optik, die in einem Schaft lösbar festgelegt ist und mit diesem ein diagnostisches Scrotoskop bildet, das mit seinem distalen Ende durch eine mit der Punktionsnadel im Scrotum zu schaffende Öffnung in das Hydroscrotum einführbar ist, um dort über die Optik zu diagnostizieren und erforderlichenfalls mit mindestens einem Hilfsinstrument zu therapieren, dadurch gekennzeichnet, daß im Schaft im Austausch gegen die als Kanüle und Dilatator ausgebildet Punktionsnadel eine Optik zur Diagnose und/oder zur Therapie lösbar festlegbar sind bzw. ist.

2. Endoskopischer Instrumentensatz nach Anspruch 1, dadurch gekennzeichnet, daß die als Kanüle zum Zu- und Abführen einer Flüssigkeit ausgebildete Punktionsnadel im Abstand vom distalen Ende mit mindestens einer nach außen gerichteten stufung versehen ist, wobei das proximalseitige Ende des kegeligen Überganges im Bereich des distalen Endes des Schaftes zu liegen kommt oder aus diesem über eine bestimmte Länge herausragt.

3. Endoskopischer Instrumentensatz nach Anspruch 1, dadurch gekennzeichnet, daß die Blickwinkel der beiden Optiken im Verhältnis zu ihren Längsachsen in einem Bereich von 5° bis 70° liegen, vorzugsweise 25° betragen, und daß die Öffnungswinkel der Optiken mindestens 50°, vorzugsweise 65° betragen.

Fig. 1

Fig. 2

Fig.3

Europäisches
Patentamt

**EUROPÄISCHER RECHERCHENBERICHT**

Nummer der Anmeldung

EP 88 11 5441

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.3) |
|---|---|---|---|
| Y | DE-C- 696 957 (GEORG WOLF GMBH) * Seite 1, Zeile 55 - Seite 2, Zeile 60; Figuren 1, 2 * | 1 | A 61 B 1/00 A 61 B 1/30 A 61 B 17/34 |
| Y | DE-A-3 025 785 (KARL STORZ) * Seite 8, Zeile 6 - Seite 12, Zeile 6; Figuren 1, 5 * | 1 | |
| A | --- | 2 | |
| A | US-A-4 254 762 (INBAE YOON) * Spalte 5, Zeile 56 - Spalte 6, Zeile 23; Spalte 5, Zeilen 3 - 12; Spalte 6, Zeilen 55 - 58; Figuren 1, 1a * | 1 - 3 | |

| | RECHERCHIERTE SACHGEBIETE (Int. Cl.3) |
|---|---|
| | A 61 B 1/00 A 61 B 17/00 |

Der vorliegende Recherchenbericht wurde für alle Patentansprüche erstellt

| Recherchenort | Abschlußdatum der Recherche | Prüfer |
|---|---|---|
| BERLIN | 16-01-1989 | WEIHS J.A. |